# EUROPEAN PATENT APPLICATION

(11) **EP 4 556 064 A1**
(43) Date of publication of application: **21.05.2025**
(21) Application number: 24210804.1
(22) Date of filing: 05.11.2024
(51) Int. Cl.: A61N 5/10

(54) **MULTI-FIELD RADIATION TREATMENT PLAN METHOD AND APPARATUS**

(30) Priority: 16.11.2023 US 202318510771
(71) Applicant: Siemens Healthineers International AG, 6312 Steinhausen (CH)
(72) Inventor: TALLINEN, Tuomas, 02600 Espoo (FI); MYLLYKOSKI, Mirko, 00380 Helsinki (FI); RUSANEN, Marko, 02940 Espoo (FI); PELTOLA, Jarkko, 04300 Tuusula (FI)
(74) Representative: Mathisen & Macara LLP

(57) **Abstract**

A control circuit 101 automatically models 201 a multi-field treatment arrangement as a single composite trajectory that includes a plurality of treatment fields to provide a single composite/multi-field treatment arrangement, and then optimizes 202 the multi-field radiation treatment plan as a function of the single composite/multi-field treatment arrangement.

## Description

### Technical Field

These teachings relate generally to optimizing a treatment plan for treating a patient's planning target volume with energy and more particularly to optimizing an energy-based treatment plan.

### Background

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

Direct aperture optimization is one approach for optimizing external beam plans having multiple overlapping treatment fields, such as co-planar arc fields. In many cases, these different fields serve to either improve plan quality by introducing more control points to be optimized, and/or help to accommodate some geometrical limit (for example, a large target) where multi-leaf collimator leaf span limitations can cause a need to split a treatment field template into multiple, somewhat or mostly overlapping fields.

Unfortunately, increasing the number of fields typically also linearly increases the optimization calculation burden and accordingly can cause optimization performance to drop. For example, by at least some prior art approaches, multi-field treatment plans are optimized by processing the fields separately. That is, each field has its own dose calculation sampling scheme for the beams and its own fluence containers, and the leaf sequence optimization is performed separately for each field. Dosimetric quality of the optimized plans can also be limited because this overlap information is not typically taken into account efficiently.

### Summary

In one aspect, the present invention provides a method to facilitate optimizing a multi-field radiation treatment plan for a multi-field treatment arrangement for a particular patient presenting a particular target volume, as defined in claim 1. Optional features are specified in the dependent claims.

In another aspect, the present invention provides an apparatus to facilitate optimizing a multi-field radiation treatment plan for a multi-field treatment arrangement for a particular patient presenting a particular target volume, as defined in claim 11. Optional features are specified in the dependent claims.

According to an embodiment of the invention, a method to facilitate optimizing a multi-field radiation treatment plan for a multi-field treatment arrangement for a particular patient presenting a particular target volume comprises automatically modeling the multi-field treatment arrangement as a single composite trajectory that includes a plurality of treatment fields to provide a single composite multi-field treatment arrangement; and optimizing the multi-field radiation treatment plan as a function of the single composite multi-field treatment arrangement.

### Brief Description of the Drawings

The above needs are at least partially met through provision of the multi-field radiation treatment plan method and apparatus described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a schematic depiction as configured in accordance with various embodiments of these teachings;
FIG. 4 comprises a schematic depiction as configured in accordance with various embodiments of these teachings; and
FIG. 5 comprises a schematic as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### Detailed Description

Generally speaking, these various embodiments serve to facilitate optimizing a multi-field radiation treatment plan for a multi-field treatment arrangement for a particular patient presenting a particular target volume. By one approach, a control circuit automatically models the multi-field treatment arrangement as a single composite trajectory that includes a plurality of treatment fields to provide a single composite/multi-field treatment arrangement, and then optimizes the multi-field radiation treatment plan as a function of the single composite/multi-field treatment arrangement.

By one approach, at least two of the plurality of treatment fields partially overlap with one another. By one approach, each of the plurality of treatment fields has a corresponding arc trajectory (where, if desired, the arc trajectories for each of the plurality of treatment fields are co-planar with one another). By one approach, at least one of the corresponding arc trajectories has a reverse (i.e., opposite) direction of another one of the corresponding arc trajectories.

By one approach, automatically modeling the multi-field treatment arrangement as a single composite trajectory comprises, at least in part, arranging each of the treatment fields of the plurality of treatment fields to have corresponding control points, wherein the control points for each of the treatment fields are co-aligned with one another and are optimized as an array of composite control points. By one approach, the control points for each of the treatment fields are co-aligned with one another such that beam directions of respective ones of the control points are at least substantially the same between the treatment fields. By one approach, and notwithstanding the co-alignment of the treatment fields with one another, a beam's eye view for a given one of the control points for a first one of the treatment fields can be different from a beam's eye view for the same given one of the control points for a second one of the treatment fields. (By one approach, the foregoing beam's eye view can be determined, at least in part, by at least one of a collimator position and a collimator angle.)

So configured, optimization can proceed more quickly as compared to prior art expectations. This result occurs, at least in part, because plans with multiple arc fields can be optimized using a smaller number of dose calculation beams and fluences that are an effective equivalent to single field plans.

Furthermore, optimization can be more efficient, at least in part because leaf positions from multiple fields can be optimized as one instance that incorporates direct dependencies between leaf positions across fields. Accordingly, these teachings can enable high-fidelity treatment modes that leverage combined modulation power of multi-field setups.

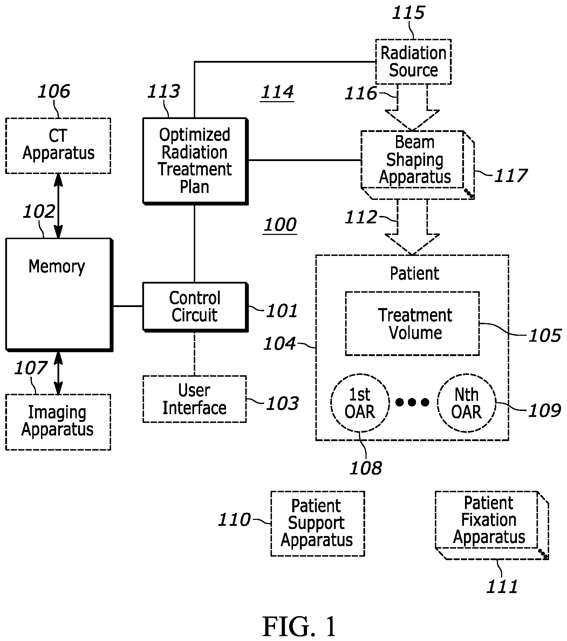

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101).

In addition to information such as optimization information for a particular patient and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as a dynamic random access memory (DRAM).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

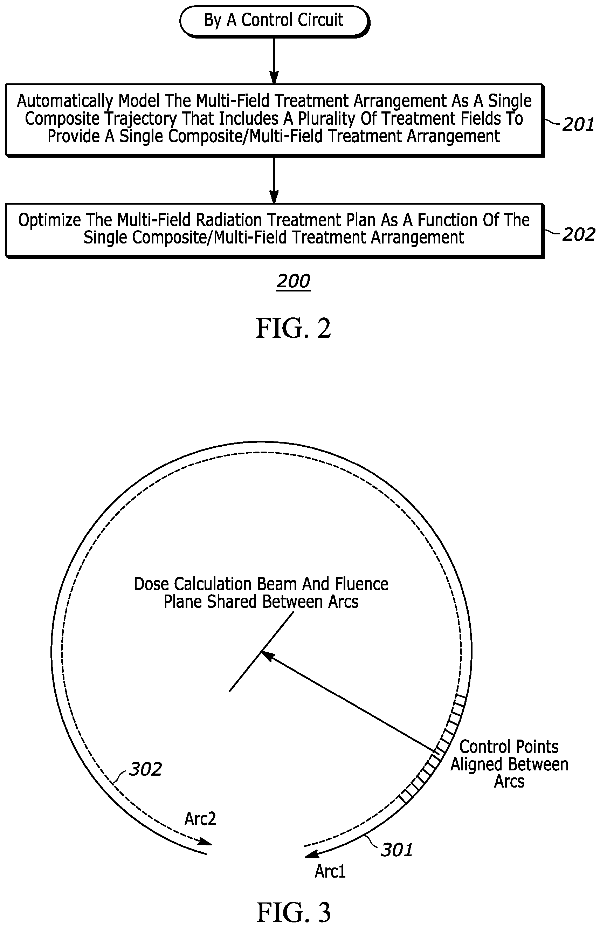

Referring now to FIG. 2, a process 200 that can be carried out, for example, in conjunction with the above-described application setting (and more particularly via the aforementioned control circuit 101) will be described. Generally speaking, this process 200 serves to facilitate generating an optimized radiation treatment plan 113 to thereby facilitate treating a particular patient with therapeutic radiation using a particular radiation treatment platform per that optimized radiation treatment plan. More particularly, these teachings facilitate optimizing a multi-field radiation treatment plan for a multi-field treatment arrangement for a particular patient who presents a particular target volume.

At block 201, this process 200 provides for automatically modeling a multi-field treatment arrangement as a single composite trajectory that includes a plurality of treatment fields to provide a single composite/multi-field treatment arrangement. By one approach, at least two of the plurality of treatment fields at least partially overlap with one another. If desired, the foregoing can comprise only partially overlapping (as versus wholly overlapping) with one another.

By one approach, each of the plurality of treatment fields has a corresponding arc trajectory. If desired, each of these arc trajectories for each of the plurality of treatment fields can be co-planar with one another. In lieu of the foregoing, or in combination therewith, at least one of the corresponding arc trajectories can have a reverse direction of movement as compared to another one of the corresponding arc trajectories.

By one approach, the above-mentioned automatic modeling of the multi-field arrangement can comprise, at least in part, arranging each of the treatment fields of the plurality of treatment fields to have corresponding control points, where the control points for each of the treatment fields are co-aligned with one another and are optimized as an array of composite control points. (Control points refer to specific radiation source positions or angles at which corresponding control point parameters reflect the state of the radiation treatment platform. Control points typically serve to help shape the radiation beam and to optimize the dose distribution to target the tumor while sparing surrounding healthy tissues.)

By one approach, the aforementioned control points for each of the treatment fields are co-aligned with one another such beam directions of respective ones of the control points are at least substantially the same between the treatment fields. By one approach, and notwithstanding the co-alignment of the treatment fields with one another, a beam's eye view for a given one of the control points for a first one of the treatment fields can be different from a beam's eye view for the same given one of the control points for a second one of the treatment fields.

By one approach, and again notwithstanding the co-alignment of the treatment fields with one another, a beam's eye view as determined, at least in part, by at least one beam shaping apparatus for a given one of the control points for a first one of the treatment fields can be different from a beam's eye view for the same given one of the control points for a second one of the treatment fields. By one approach, the aforementioned beam's eye view can be determined, at least in part, by at least one of a collimator position and/or a collimator angle.

At block 202, the control circuit 101 can then optimize the multi-field radiation treatment plan as a function of the aforementioned single composite/multi-field treatment arrangement. Various optimization approaches are known in the art. As the present teachings are not overly sensitive to any particular selections in these regards, further elaboration in these regards is not provided here for the sake of brevity.

In some direct aperture optimization methods, the full control point sequence representing, for example, an arc field is split into smaller groups of consecutive control points, each control point being associated with a dose calculation beam, fluence plane, and leaf optimization instance (comprising a so-called dose calculation sector). Instead of separate sets of sectors for each field, the present teachings can provide that co-aligned control points from multiple fields are together in a single set of sectors. This approach reduces the necessary computational load as dose from multiple fields can be calculated on a shared set of fluences and beam directions. Also, leaf sequences of multiple fields can be optimized jointly on a single set of fluences, which means that the leaf sequencing algorithm (typically a major sub-component in direct aperture optimization) can directly and implicitly optimize leaf apertures of multiple fields for optimal total fluence.

Additionally, by one approach, leaf sequence solutions for each field within a sector can be controlled using a weight map per field (using, for example, percentage values of between 0 and 100) that can be overlaid over the target fluence map of the sector. This can be used for creating a ramp-down/ramp-up field fluence at the vicinity of the actual field borders, thus avoiding a hard cut at the border where one field contribution ends and another field contribution starts. Such a weight map can also be used to guide the different fields to optimize different parts of the same fluence by using it as a mask per field.

The resultant optimized multi-field radiation treatment plan can serve to administer therapeutic radiation to the particular patient using the corresponding radiation treatment platform 114.

Further details that comport with these teachings will now be presented. It will be understood that the specific details of these examples are intended to serve an illustrative purpose and are not intended to suggest any particular limitations with respect to these teachings.

As described above, a multi-field setup can be modeled as a single composite arc or trajectory. By one approach, this can comprise splitting a user-defined arc template into multiple arc fields, or by grouping user-defined co-planar arc fields. In this illustrative example, fields are assumed to be aligned such that beam directions of their control points are equal between fields, but beam's eye views defined by collimator positions and collimator angle are allowed to be different from one another.

Arc rotation directions can also be opposite one another. For example, a plan with a wide total field opening can be produced by two or more co-planar arcs with side-by-side beams' eye views and back-and-forth gantry revolutions. FIG. 3 presents such a dual-arc scenario example, with a first arc 301 have a clockwise direction and a second arc 302 having a counter-clockwise direction. This figure further illustrates two co-planar arcs 301 and 302 that are aligned by their control points. In particular, each control point of the first arc 301 has a corresponding control point in the second arc 302 (and vice versa) with the same gantry position, couch angle, and isocenter. In this illustrative example, dose calculation sectors are shared, with single beam and fluence plane being mapped to the corresponding sectors of control points from both arcs 301 and 302.

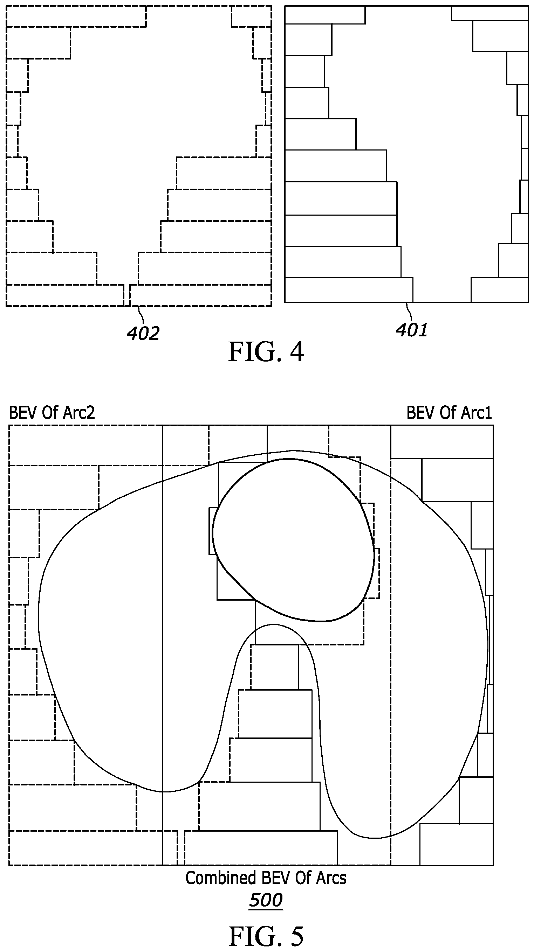

FIG. 4 presents an illustrative example of a beam's eye view 401 at a given point for the aforementioned first arc 301 and a beam's eye view 402 for the corresponding point for the aforementioned second arc 302. FIG. 5 then presents the combination 500 of those two beam's eye views 401 and 402. More specifically, this figure helps to illustrate that apertures from overlapping beam's eye views of two arcs can be optimized together for a single target fluence on a shared fluence map, thereby producing direct feedback between the leaf sequences of both arcs.

Those skilled in the art will recognize that a wide variety of modifications, alterations, and combinations can be made with respect to the above-described embodiments without departing from the scope of the invention. As one example in those regards, partitioning of total beam's eye views for different fields, and optionally generation of field specific weight maps, can be automated based on, for example, size of the beam's eye views and/or geometry of target volume projections. As another example, in some definitions complex arc trajectories, including, for example, overlapping back and forth gantry rotations, may be presented technically as a single field. In the above teachings, such geometry can be assumed as multi-field configuration if there are multiple field sections with overlapping beam's eye views. Accordingly, such modifications, alterations, and combinations are to be viewed as being within the ambit of the inventive concept.

## Claims

1. A method to facilitate optimizing a multi-field radiation treatment plan for a multi-field treatment arrangement for a particular patient presenting a particular target volume, the method comprising:
by a control circuit:
automatically modeling the multi-field treatment arrangement as a single composite trajectory that includes a plurality of treatment fields to provide a single composite/multi-field treatment arrangement;
optimizing the multi-field radiation treatment plan as a function of the single composite/multi-field treatment arrangement.

2. The method of claim 1 wherein at least two of the plurality of treatment fields partially overlap with one another.

3. The method of claim 1 or 2 wherein each of the plurality of treatment fields has a corresponding arc trajectory.

4. The method of claim 3 wherein the arc trajectories for each of the plurality of treatment fields are co-planar with one another.

5. The method of claim 3 or 4 wherein at least one of the corresponding arc trajectories is a reverse direction of another one of the corresponding arc trajectories.

6. The method of any one of claims 1 to 5 wherein automatically modeling the multi-field treatment arrangement as a single composite trajectory comprises, at least in part, arranging each of the treatment fields of the plurality of treatment fields to have corresponding control points, and wherein the control points for each of the treatment fields are co-aligned with one another and optimized as an array of composite control points.

7. The method of claim 6 wherein the control points for each of the treatment fields are co-aligned with one another such that beam directions of respective ones of the control points are at least substantially the same between the treatment fields.

8. The method of claim 7 wherein notwithstanding the co-alignment of the treatment fields with one another, a beam's eye view for a given one of the control points for a first one of the treatment fields can be different from a beam's eye view for the same given one of the control points for a second one of the treatment fields.

9. The method of claim 7 wherein notwithstanding the co-alignment of the treatment fields with one another, a beam's eye view as determined, at least in part, by at least one beam shaping apparatus for a given one of the control points for a first one of the treatment fields can be different from a beam's eye view for the same given one of the control points for a second one of the treatment fields.

10. The method of claim 9 wherein the beam's eye view is determined, at least in part, by at least one of a collimator position and a collimator angle.

11. An apparatus to facilitate optimizing a multi-field radiation treatment plan for a multi-field treatment arrangement for a particular patient presenting a particular target volume, the apparatus comprising:
a control circuit configured to:
automatically model the multi-field treatment arrangement as a single composite trajectory that includes a plurality of treatment fields to provide a single composite/multi-field treatment arrangement;
optimize the multi-field radiation treatment plan as a function of the single composite/multi-field treatment arrangement.

12. The apparatus of claim 11 wherein at least two of the plurality of treatment fields partially overlap with one another.

13. The apparatus of claim 11 wherein each of the plurality of treatment fields has a corresponding arc trajectory.

14. The apparatus of claim 13 wherein:
the arc trajectories for each of the plurality of treatment fields are co-planar with one another; and/or
at least one of the corresponding arc trajectories is a reverse direction of another one of the corresponding arc trajectories.

15. The apparatus of any one of claims 11 to 14 wherein the control circuit is configured to perform the method of any one of claims 6 to 10.
